# EUROPEAN PATENT SPECIFICATION

(11) **EP 1 330 253 B1**
(45) Date of publication and mention of the grant of the patent: **11.08.2004**
(21) Application number: 00990873.2
(22) Date of filing: 20.10.2000
(51) Int. Cl.: A61K 35/22, A61P 43/00

(54) **USE OF BIOACTIVE FRACTION FROM COW URINE DISTILLATE ('GO MUTRA') AS A BIO-ENHANCER OF ANTI-INFECTIVE, ANTI-CANCER AGENTS AND NUTRIENTS**
VERWENDUNG DER BIOAKTIVEN FRAKTION AUS DEM URINDESTILLAT DER KUH ("GO MUTRA") ZUR VERSTÄRKUNG DER WIRKSAMKEIT VON ANTI-INFEKTIVA, ANTI-KREBSMITTELN UND ERNÄHRUNGSMITTELN
UTILISATION D'UNE FRACTION BIO-ACTIVE PROVENANT D'UN DISTILLAT D'URINE DE VACHE ( GO-MUTRA ) EN TANT QUE BIO-ACTIVATEUR D'AGENTS ET D'ELEMENTS NUTRITIFS ANTI-INFECTIEUX ET ANTICANCEREUX

(43) Date of publication of application: 30.07.2003
(73) Proprietor: Council of Scientific and Industrial Research, New Delhi 110 001 (IN)
(72) Inventor: KHANUJA, S.P.S., Ctl Inst. Med. & Aromatic Plants, Lucknow 226 010, Uttar Pradesh (IN); KUMAR, S, Ctl Inst. of Med. & Aromatic Plants, Lucknow 226 010, Uttar Pradesh (IN); SHASANY, A.K, Cent.Inst. of Med. & Aromatic Plants, Lucknow 226 010, Uttar Pradesh (IN); ARYA, J.S., Cent. Inst. of Med. & Aromatic Plants, Lucknow 226 010, Uttar Pradesh (IN); DAROKAR, M.P., Cent.Inst. of Med. & AromaticPlants, Lucknow 26 010, Uttar Pradesh (IN); SINGH, M., Cent. Inst. of Med. & Aromatic Plants, Lucknow 226 010, Uttar Pradesh (IN); SINHA, P, Cent.Inst. of Med. & Aromatic Plants, Lucknow 226 010, Uttar Pradesh (IN); AWASTHI, S., Cent.Inst. of Med. & Aromatic Plants, Lucknow 226 010, Uttar Pradesh (IN); GUPTA, S.C., Cent.Inst. of Med. & Aromatic Plants, Lucknow 226 010, Uttar Pradesh (IN); GUPTA, V.K., Cent. Inst of Med. & Aromatic Plants, Lucknow 226 010, Uttar Pradesh (IN); GUPTA, M.M., Cent.Inst. of Med. & Aromatic Plants, Lucknow 226 010, Uttar Pradesh (IN); VERMA, R.K., Cent.Inst. of Med. & Aromatic Plants, Lucknow 226 010, Uttar Pradesh (IN); AGARWAL, S., Cent.Inst. of Med. & Aromatic Plants, Lucknow 226 010, Uttar Pradesh (IN); MANSINGHKA, S.B., Cent. Inst. Med. & Aroma. Plants, Lucknow 226 010, Uttar Pradesh (IN); DAWLE, S.H., Cent. Inst. of Med. & Aromatic Plants, Lucknow 226 010, Uttar Pradesh (IN)
(74) Representative: Wain, Christopher Paul
(86) International application number: PCT/IN2000/000106
(87) International publication number: WO 2002/032436

(56) References cited:
- US-A- 3 248 295
- OLUSI S O ET AL: "EVIDENCE FOR COMPLEMENT ACTIVATION FOLLOWING THE ORAL ADMINISTRATION OF COWS URINE CONCOCTION IN RATS." AFR J MED MED SCI, (1978) 7 (2), 79-84. , XP000996728
- CHEMICAL ABSTRACTS, vol. 121, no. 7, 1994 Columbus, Ohio, US; abstract no. 74040b, page 142; XP002167367 & VAN GINKEL L.A. ET AL: "The certification of the individual stilbene mass concentrations in lyophilized bovine urine CRMs" COMM. EUR. COMMUNITIES, vol. EUR15027, 1994, page eur

## Description

### Technical Field

The invention relates to cow urine distillate for use as an activity enhancer and availability facilitator for bioactive molecules including anti-infective and anti-cancer agents. The molecules which express any activity in form of either inhibiting or promoting a biological function have been referred in this invention as bioactive molecule e.g. antibiotics, drugs, nutraceuticals, cardiovascular, hepatoprotective, neuro-tonics *etc.* The present invention has direct implication in drastically reducing the dosage of antibiotics, drugs and anti-cancer agent while increasing the efficiency of absorption of bioactive molecules.

### Background art

In Ayurveda cows urine is suggested for improving general health. But it is never scientifically tested for any utility alone. The applicants have developed the curiosity about this component in the preparations and asked many questions to them; whether the component cow urine is having any activity by itself or it does not have any activity but enhance the activity of other components in the preparations? What are the components present in the urine of cow? Whether the urine contains microorganism, which are beneficial? Whether the degradation products from the urine are beneficial? To answer these questions the applicant obtained "Kamadhenu Arka" the urine distillate from "Go-Vigyana Anusandhana Kendra" Nagpur, India. This is the urine distillate suggested for drinking to improve the general health and sold and distributed in different size bottles. The applicants tested the urine on Luria agar and broth in sterile condition and did not notice any growth of microorganism. To test whether this is inhibitory to growth of different microorganism, *Escherichia coli* and *Mycobacterium smegmatis* were grown at different temperatures ranging from 20 to 40°C in presence and in absence of the cow urine distillate, no significant difference in the colony count is noticed. Surprisingly, the same distillate enhanced the antibiotic action on these bacteria leading to this invention. The novelty of the invention lies in the fact revealed through precise experimentation that the enhancement action and its effectiveness is achievable only in the range of concentration which is literally in nano to micro molar levels. And when a higher concentration / dosage is used in the formulation or combinations the activity(ies) do not appear. That should be the reason for non-detection such a valuable potential of cow urine (*Go-mutra*)*.* From million doses of annual antibiotics consumption goes waste as these could not be utilized or targeted to the infective organisms effectively due to various factors like efficient absorption, transportation to the target site, retention time, operation of efflux pump, metabolism *etc.* Thus, large portions of the drugs we apply are wasted and only a minuscule percentage is being targeted to the infective microbes. Also, the unutilized drug / antibiotic amount remains as a load in the body and environment acting as a selection pressure to facilitate emergence of drug resistance in parasites and their predominance, ultimately leading to failure of antibiotics against resistant infections. This also is responsible for side effects, illness and reduction in life expectancy being more acute in the older population. One of the ways, which has been feasible to reduce drug dosage, has been synergism between two therapeutic agents. However, if both have the antibiotic property, all the problem of continued selection pressure on microbes is likely to continue. So, the applicants thought of utilizing cow urine, which is not microbicidal but when present with a drug or active molecule, enhance its activity and availability (bioenhancers). This way, the selection pressure will be counter-balanced simultaneously reducing the dosage of antibiotics or drugs for minimizing the side effects, which has also high commercial importance.

The present invention was the result of planned experiments to provide a novel composition for improving activity and bioavailability of antibiotics, drugs and other molecules using 'cow urine distillate' in different formulations.

The bioavailability of nutrients and enhancement antibiotics effect is relevant to human, plant as well as animal health and thus the compositions and methods of the invention are also intended to be used in agriculture and veterinary practice.

Cow's urine (*Go-mutra*) can be considered as the most effective animal origin substance/secretion with the capacity of general health improvement but it does need substantiation through scientific experimentation. Thus, the applicants considered it worthwhile to scientifically look at this and define the molecular basis of the values through *in vitro* and *in vivo* assays. The applicants in the first instance probed whether it contained any drug facilitator elements since such a property would make it a highly useful natural substance. In recent days, use of 'piperine' as a bioavailability enhancer has been described (United States Patents 5,616,593 and 5,972,382). Till today thus, the known bioavailability enhancer documented is piperine and a series of inventions related to this compound have been described in the following prior arts.

### Disclosure of the invention

The main objective is to provide new use of the bio- active fraction as a bio-enhancer and as a bioavailability facilitator.

In another objective of the invention is to provide a composition for improving activity and bioavailability of antibiotics, drugs and other molecules using active fraction from cow urine distillate.

Still another objective of the invention is to provide a process for the extraction of the active fraction form the cow urine.

### Summary of the invention

The invention relates to a known abundantly available cow urine distillate as an enhancer of antibiotic action on the target. The molecule of invention helps in the absorption of antibiotics across the cell membrane in animal cells, gram positive and gram negative bacteria. Similar activities can also be obtained by using the distillate of the urine of cow at 40-50°C and from the concentrate, which is lyophilized and dissolved for further use. Further the urine distillate from buffalo, camel deer provide similar activity of bioavailability.

### Brief description of the accompanying drawing

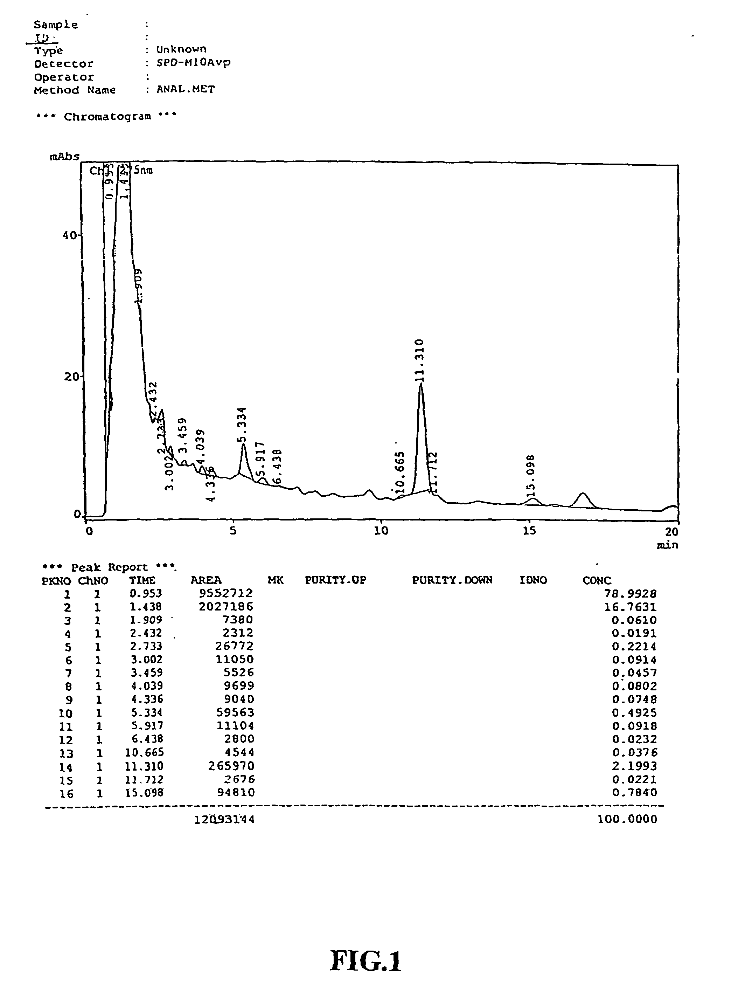

Fig. 1 represents HPLC characters of cow urine (*Go-mutra*) distillate

### Detailed description of the invention

Our emphasis here was to search a plentifully available material with bioenhancing action of higher potency. Additionally, a property that the applicants searched was the bioenhancement ofa scarcely available anti-cancer natural agent 'taxol' (peclitaxel) which is produced in microscopic amounts by the Yew tree (*Taxus* spps.) and hence is always a limited molecule in availability. Cow urine distillate enhanced the killing activities of different antibiotics on bacteria. More important was the obvious enhancement in the cell division inhibitory activity against the breast cancer cell line MCF-7.

In an embodiment of the present invention a pharmaceutical composition comprising an effective amount of cow urine distillate as a bioavailability facilitator and a pharmaceutically acceptable additives selected from anticancer compounds, antibiotics, drugs, therapeutic and nutraceutic agents, ions and similar molecules which are targeted to the living systems.

In another embodiment, the cow urine distillate is to be used as bioavailability facilitator for anticancer therapy directly or in combination with anticancer molecules.

In still another embodiment, the cow urine distillate is to be used in antifungal therapy for fungal infections.

In yet another embodiment, the antifungals are azoles, clotrimazole, mystatin, amphotericin and similar materials.

In yet another embodiment, fungi covering infections are mycetial, candida, yeast or other fungicidal compounds.

In still another embodiment, the cow urine distillate is to be used in TB therapy including multi drug resistant tuberculosis in combination with isoniazid and other anti-tubercular agents.

In yet another embodiment, the bioavailability facilitator helps in transferring the compound across the membrane and for better effectivity on the target site.
In yet another embodiment, the antibiotics are Quinolones, fluoroquinolones like Nalidixic acid and others like Rifampicin, Tetracycline, ampicillin and similar compounds.

In yet another embodiment, the antibiotics, ions and similar compounds are isoniazid and hydrogen peroxide.

In yet another embodiment, the bioavailability facilitator helps the antibiotics and other molecules to act better on the target by increasing the effectivity.

In yet another embodiment, the living system may be bacteria, fungi or any living cells.

In yet another embodiment, the anti bacterial agents are selected from the group comprising Quinolones, Rifampicin, Tetracycline and ampicillin.

In yet another embodiment, the cow urine (*Go-mutra*) distillate is in the range between 0.001 µl/ml to 100 µl/ml.

In yet another embodiment, the lyophilized active fraction used is in the range between 0.1 µg/ml to 100 µg/ml.

In still another embodiment, the bioactive fraction enhances the activity of anti-bacterial agents, anti-cancer agents and anti-tuberculosis agents from 2 to 80 folds.

In yet another embodiment, the bioactive fraction enhances the activity of anti-bacterial agents from 2 to 80 folds.

In yet another embodiment, the anti bacterial agent is an anti-tuberculosis agent selected from isoniazid, pyrazinamide and other similar compounds.

In yet another preferred embodiment, the bioactive fraction enhances the activity of anti-tuberculosis agents from 2 to 20 folds.

In yet another embodiment of the invention, the anti-cancer agent is selected from group consisting of Paclitaxel (Taxol).

In yet another preferred embodiment of the invention, the bioactive fraction enhances the activity of anti-cancer agents from 2 to 20 folds.

The methodology followed by us for this screening included specifically designed bioassays as described below. The bacterial and fungal strains used in this invention were acquired commercially from Institute of Microbial Technology (IMTECH), Chandigarh which possessed corresponding properties of the ATCC strain mentioned.

### 1. Assay for bio-enhancement of anti-infective agents

a) The minimum inhibitory concentration (MIC) of antibiotic is determined against *Escherichia coli* (equivalent of ATCC 10536), *Bacillus subtilis* (equivalent of ATCC 6015) and *Mycobacterium smegmatis* (equivalent of ATCC 10231) in broth and disc diffusion assay.
b) The antibiotics agents at concentrations 1/4, 1/3, 1/2 and equal to MIC are added alone and in combination with the test compound at varying concentrations on disc and in broth to evaluate the comparative inhibition.
c) These combination showing significant advantage or higher activity than antibiotic alone in terms of enhanced inhibition of bacterial growth (large inhibition zone in disc diffusion and affectivity of lower concentration in broth assay) are picked up for future testing.
d) In broth assay the activity is quantified by counting number of viable cells in a given treatment and converted in fold enhancement by combination compared to antibiotic / drug alone in the killing percentage of cells.
e) The pre-treatment assay followed to determine whether the compound is required along with antibiotic to enhance its activity or even its withdrawal after treatment or prior to antibiotic treatment would benefit. For this, the cells are treated with compound for 4 to 8 hours and then washed free of it by centrifugation and washing in sterile water. This was followed by treatment with antibiotic as in steps b to d.

### 2. Assay for bio-enhancement of anti-cancer agents

a) MCF-7 (Breast cancer commercial cell line obtained from National Center for Cell Sciences (NCCS), Pune) is inoculated at a density of about 0.1 X 10⁶ cells in MEM medium in the wells of 24 well plate.
b) This is replaced with fresh medium after 18 hours in each well.
c) The test component (s) is added at desired concentrations in different wells just after the medium replacement.
d) Observations are recorded on the cell count after 36 hours for which the allowing steps are required.
   i. The medium is removed from the wells.
   ii. The wells are rinsed with 1ml PBS (Phosphate buffer saline).
   iii. To each well 500 µl of freshly prepared trypsin (0.1% in PBS) solution is added.
   iv. Trypsin solution is removed after 30 seconds and the plate is gently tapped till the cells are released from the plate surface.
   v. Fresh 1 ml of MEM growth medium is added and agitated with a pipette to obtain a cell suspension.
   vi. 10 µl of cell suspension is taken on the haemocytometer and a cover glass is placed over the counting chamber.
   vii. The number of viable cells is counted in 5 big squares and the readings are taken from 5 microscopic fields to determine the average.
   viii. The cell count (titer per ml) in the original sample is then calculated as average count X 10³.

### Composition of Minimum Essential Medium (MEM): 100 ml

| | |
|---|---|
| MEM powder (Sigma -Aldrich, USA) = | 0.96 g |
| HEPES Buffer (Sigma -Aldrich, USA) = | 0.26 g |
| Sodium Bicarbonate = | 0.22 g |
| Penicillin G = | 10 mg |
| Streptomycin = | 20 mg |
| Gentamycin = | 5 mg |
| Foetal Calf Serum = | 15 ml |
| Foetal Calf Serum = | 15 ml |
| Distilled water = | 85 ml |

### 3. Bioavailability tests through biological membrane

a) Specially designed U-tubes of glass consisting of two components (opposite-L type) were used in which one open end of an L-shaped was tapered to fit within the untapered end of the other L-tube (Fig 1).
b) The membrane of goat gut (initial part) was stretched and fixed to act as the barrier between the two ends such that by joining the two L-tubes, a U-tube was made.
c) Sterile distilled water was then filled in both the sides to equal height/level.
The antibiotic/compound was added to the donor tube (tapered) and through spectrophotometer, the transfer of molecule was observed using UV and visible absorption maxima of the respective molecules by taking the OD at defined wavelengths.

### Examples

In the next step of elucidation of the enhancer action, the applicants experimented with the killing activities of different antibiotics against the bacteria singly and in combination with the test component (cow urine distillate) following the method described above. These experiments are being described in the following examples. When the bacteria were grown in presence of the compound as such no significant killing was observed. In all the experiments the cow urine distillate concentration was kept at 1 µl/ml, unless it is specifically mentioned.

### Example 1. Cow urine distillate mediated enhancement in the killing action of antibiotics against Gram-negative bacterium Escherichia coli.

**Table 1:**

| Antibiotics | Concentration µg/ml | Survival fraction of viable cells upon treatment with antibiotic alone | Survival fraction of viable cells upon treatment with amlibiotic + cow urine distillate combination | * Fold enhancement in antibiotic activity |
|---|---|---|---|---|
| Rifampicin | 10 | 0.86 | 0.17 | 5.0 |
| Rifampicin | 30 | 0.05 | 0.007 | 7.1 |
| Ampicillin | 4 | 1.11 | 0.60 | 1.85 |
| Ampicillin | 6 | 0.09 | 0.02 | 4.50 |
| Ampicillin | 8 | 0.05 | 0.01 | 5.00 |

It was calculated as Survival fraction of viable cells upon treatment with antibiotic and cow urine distillate in combination / Survival fraction of viable cells upon treatment with antibiotic alone

### Example 2. Cow urine distillate mediated enhancement in the killing action of antibiotics against Gram-positive bacterium Bacillus subtilis.

**Table 2:**

| Antibiotics | Concentration µg/ml | Survival fraction of viable cells upon treatment with antibiotic alone | Survival fraction of viable cells upon treatment with antibiotic + cow urine distillate combination | * Fold enhancement in antibiotic activity |
|---|---|---|---|---|
| Rifampicin | 0.005 | 1.1 | 0.28 | 5.5 |
| Rifampicin | 0.05 | 0.03 | 0.01 | 3.0 |
| Ampicillin | 0.1 | 1.00 | 0.3 | 3.3 |
| Ampicillin | 0.5 | 0.18 | 0.06 | 3.0 |

### Example 3. Cow urine (Go-mutra) distillate mediated enhancement in the killing action of antibiotics against bacterium Mycobacterium smegmatis

**Table 3:**

| Antibiotics | Concentration µg/ml | Survival fraction of viable cells upon treatment with antibiotic alone | Survival fraction of viable cells upon treatment with antibiotic + cow urine distillate combination | * Fold enhancement in antibiotic activity : |
|---|---|---|---|---|
| Rifampicin | 0.05 | 0.008 | 0.0001 | 80 |
| Rifampicin | 0.1 | 0.006 | 0.0036 | 1.5 |
| Ampicillin | 0.5 | 0.07 | 0.006 | 11.6 |

From the above experiments it was deduced that the potency of the antibiotic is increased when applied along with cow urine distillate.

### Example 4: Cow urine distillate mediated enhancement in the killing action of izoniazid and hydrogen peroxide against oxyR mutant of bacterium Escherichia coli. The cow urine distillate concentration is 0.001 µl/ml

**Table 4:**

| | Concentration | Survival fraction of viable cells upon treatment with isoniazid/H₂O₂ alone | Survival fraction of viable cells upon treatment with a isoniazid/H₂O₂ + cow urine distillate combination | Fold enhancement in isoniazid/H₂O₂ activity |
|---|---|---|---|---|
| Isoniazid | 250µg/ml | 7.5 x10⁷ | 0.99 x10⁷ | 7.5 |
| Hydrogen peroxide (H₂O₂) | 0.003 %v/v | 7.14 x10⁷ | 1.2 x10⁷ | 5.9 |

The *oxyR* gene is required for the induction of a regulon of hydrogen peroxide-inducible genes in *Escherichia coli* (Christman M F, Storz G and Ames BN (1989) Oxy R, a positive regulator of hydrogen peroxide-inducible genes in *Escherichia coli* and *Salmonel'* *typhimurium*, is homologous to a family of bacterial regulatory proteins (Proc. Natl. Acad. Sci. (USA). 86:3484-3488.). The mutants of these genes are sensitive to drugs like isoniazid and hydrogen peroxide, which produce free radicals, damaging the cellular systems. So the killing activities of these compounds are increased by 5 to 8 folds by cow urine.

### Example 5: Cow urine distillate mediated enhancement in the activity of anti cancerous compounds. The cow urine distillate concentration is 1 µl/ml

**Table 5:**

| Taxol Concentration µg/ml | Initial titre of viable cells | Final titre of viable cells upon treatment with taxol alone | Final titre of viable cells upon treatment with taxol + cow urine distillate |
|---|---|---|---|
| 0.001 | 0.9 X 10⁻⁶ | 0.059 X 10⁶ | 0.039 X 10⁶ |
| 0.005 | 0.9 X 10⁻⁶ | 0.042 X 10⁶ | 0.032 X 10⁶ |
| 0.01 | 0.9 X 10⁻⁶ | 0.036 X 10⁶ | 0.012 X 10⁶ |

The applicants observed similar results of enhancement in the animal cell culture (cancerous cell line MCF-7 obtained from National Center for Cell Sciences (NCCS), Pune), in which the killing action of anti cancerous chemical 'Taxol' is increased. The components of cow urine distillate in our study help in transferring other compounds across the membrane thereby increasing the absorption in, irrespective of bacteria, animal and plant cell. This in-turn has immense importance for absorption of the drugs, pharmaceuticals, nutraceutical and other related compounds and ions by the cells.

### Example 6: Bioenhancement of antifungal agent clotrimazol by bioactive Fraction Gm-IV. The concentration of the active fraction is kept at 10 µg/ml.

**Table 6**

| Treatment Treatment | Minimum inhibitory Concentration (MIC) in µg/ml | Fold enhancement |
|---|---|---|
| Clotrimazol | 4.40 | 0.0 |
| Clotrimazol + Gm IV | 0.88 | 5.0 |

In other observations the compound cow urine distillate enhances the transport of antibiotics e.g. Rifampicin, Tetracycline, Ampicillin across the gut as well as artificial membrane. The enhancement in transport is approximately 2 to 7 folds.

Great emphasis now is being laid towards quality assurance of crude drugs from alternative sources widely used in the Indian system of medicine. The present invention enlarges the scope and use of the cow urine distillate in therapeutical and nutraceutical application.

### Example 7

**Development of powder form:** For enhancing the utility and convenience of application of cow urine (*Go-mutra*), the applicants further fractionated to reach a solid form which is also free of the typical smell of cow urine distillate that it is more readily acceptable to the humans. For this purpose the urine distillate was fractionated as described by the following procedure:

### Fractionation of a white crystalline solid from cow urine.

Step 1: Cow urine is collected aseptically in the stainless steel container directly from the cow, which is maintained in hygienic environment
Step 2: Fifteen liters of cow urine is distilled continuously at 40 - 50 °C in glass distillation apparatus to obtain 10 - 12 liters of the distillate in 16 to 18 hours.
Step 3: The distillate is packed in surface sterilized plastic or glass container for further use
Step 4: 200 ml of cow urine distillate was mixed with half the volume of methanol and extracted with hexane.
Step 5: The hexane fraction (Gm-I) was lyophilized and tested for similar activity as that of cow urine *(Go-mutra)*.
Step 6: The aqueous fraction was extracted with ethyl acetate and the ethyl acetate fraction (Gm-II) was lyophilized and tested for similar activity as that of cow urine (*Go-mutra*).
Step 7: Further, aqueous fraction containing white precipitate was extracted with butanol and the butanol fraction (Gm-III) having pale yellow precipitate was lyophilized and tested for similar activity as that of cow urine (*Go-mutra*).
Step 8: The remaining aqueous fraction containing white crystalline precipitate (Gm-IV) was dried and tested for similar activity as that of cow urine (*Go-mutra*).

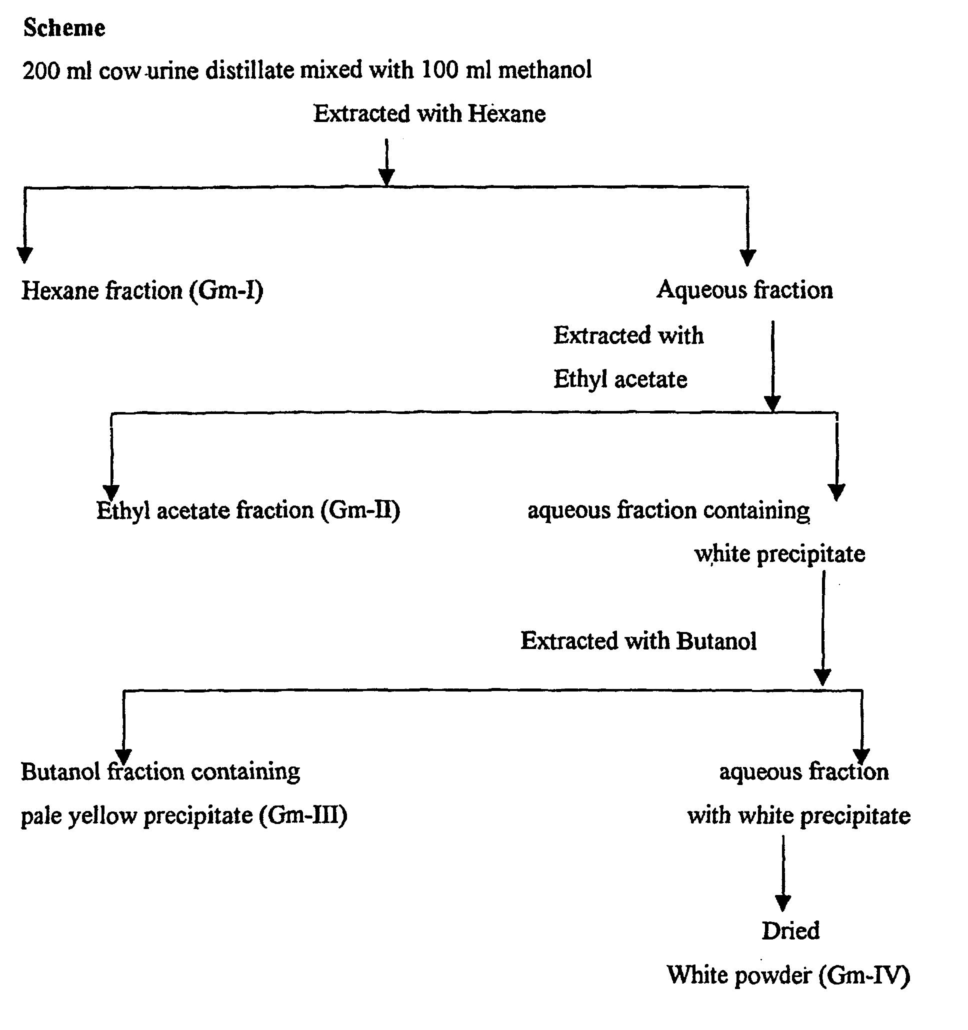

The white powder (Gm-IV) that was obtained in the range of 10 to 20 grams per 100 ml of the distillate showed all the above activities as described for cow urine distillate at concentration 0.001 to 10 µg/ml, with much more stability and being devoid of the unpleasant smell and hence was used as the advanced product of the invention. The novelty of the invention lies in the fact revealed through precise experimentation that the enhancement action and its effectiveness is achievable only in the range of concentration which is literally in nano to micro molar levels. And when a higher concentration / dosage is used in the formulation or combinations the activity(ies) do not appear.

### Physical characters of the Gm-IV fraction

Coior: White
Physical state: Solid Crystalline
Solubility: Water-soluble and mixture containing water
Melting point: Above 400°C
Specific Gravity: 1.006
RF value in methanol: Chloroform (50: 50) phase: 0.65

### HPLC Characterization of cow urine (Go-mutra) distillate

HPLC was performed on LC-8A Shimadzu HPLC with mobile phase water: acetonitrile (80:20), flow rate 1.0ml/min, UV detection at 275 nm and C-18 E MERCK (150 X 4 mm) column. Two major peaks (retention time 5.334 and 11.310 min) observed in the profile of cow urine (*Go-mutra*) distillate (Figure 1).
Further characterization to test the chemical nature of the compound was performed through Feigl's test (In: E Stahl, Thin Layer Chromatography) which was positive indicating the presence of glycoside or sugar.
The novelty of the invention is that from cow urine (*Go-mutra*) distillate, a stable solid fraction could be isolated which is water soluble and devoid of the urine smell and can directly be used in any formulation.
The fraction Gm-IV also enhances the transport of antibiotics and vitamins across the mammalian gut membrane. The example describing the enhanced transport of rifampicin by the fraction Gm-IV is given below.

### Example 8: Fraction Gm-IV of cow urine (Go-mutra) distillate mediated enhancement in the bioavailability across the biological membrane (Rifampicin, 1mg/ml and fraction Gm-IV, 1.0µg/ml).

**Table 7:**

| Compound(s) in the donor tube | Wave length (nm) | OD measured as Absorbance (specific to the compound maxima) across the membrane in receiving tube after | | | | | |
|---|---|---|---|---|---|---|---|
| | | 1 hr | 2 hr | 3 hr | 4hr | 5 hr | 6 hr |
| Rifampicin | A₃₄₀ | 0.0097 | 0.0214 | 0.0334 | 0.0771 | 0.0858 | 0.0910 |
| | A₄₇₅ | 0.0177 | 0.0284 | 0.0309 | 0.0412 | 0.0484 | 0.0496 |
| Rifampicin + Gm-IV | A₃₄₀ | 0.0525 | 0.0961 | 0.1353 | 0.1639 | 0.1919 | 0.1989 |
| | A₄₇₅ | 0.0502 | 0.0904 | 0.0793 | 0.0966 | 0.1157 | 0.1183 |

As cow urine (*Go-mutra*) distillate and the fraction Gm-IV, both shows enhanced membrane permeability by enhancing the antibiotics across the semi-permeable membrane and mammalian gut membrane, it can be assumed the above substances can be used for enhancing intestinal transport and transport of molecules across membranes of various biological functions including urinary / renal systems.

In all the experiments 1 to 5, the enhancing activity of the lyophilized product of the invention is found to have same enhancing activity like that of the distillate.

The invention can further be explained as follows:
1. The sample shows enhancement of bioavailability of rifampicin (antibiotic) and Vitamin B-12 across the mammalian gut membrane (Goat intestine was used) within 2 hours and it keeps increasing upto 4 hours.
2. It shows clear inhibition of ascorbic acid action to prevent oxidation of cut apple indicating that it probably enhances the isoniazid (INH) by oxidative mechanism that synergises the drug action of INH.
3. The distillate showed enhancement action for INH even at 10-50 thousand-fold dilution in the final volume of culture.
4. Still more interesting observation is that at 1 µl/ml the distillate showed enhancement in the activity of taxol by at least 5 folds. Further experiments in this direction have been taken up on priority.

## Claims

1. A pharmaceutical-composition comprising an effective amount of cow urine distillate as a bioavailability facilitator and pharmaceutically acceptable additives selected from anticancer compounds, antibiotics, drugs, therapeutic and nutraceutic agents, ions and similar molecules which are targeted to the living systems.

2. A composition as claimed in claim 1 wherein, the cow urine distillate is used as a bioavailabilty facilitator for anticancer therapy directly or in combination with anticancer molecules; in TB therapy including multi drug resistant tuberculosis in combination with isoniazid and other anti-tubercular agents; or in antifungal therapy for fungal infections.

3. A composition as claimed in claim 1 wherein, the bioavailabilty facilitator helps in transferring the compound across the membrane and for better effectivity on the target site; and/or helps the antibiotics and other molecules to act better on the target by increasing the effectivity; and/or enhances membrane permeability of molecules, drugs across the semi-permeable membrane and mammalian gut membrane, and is used for enhancing intestinal transport and transport of molecules across membranes of various biological functions.

4. A composition as claimed in claim 1 wherein the antibiotics are but not limited to fluoroquinolones like nalidixic acid and others like rifampicin tetracycline and ampicillin.

5. A composition as claimed in claim 1 wherein, the antibiotics, ions and similar compounds are isoniazid and hydrogen peroxide.

6. A composition as claimed in claim 1 wherein, the antifungal agents are azoles, clotrimazole, mystatin, amphotericin and similar materials.

7. A composition as claimed in claim 1 wherein fungi covering infections are mycetial, candida, yeast or other fungicidal compounds.

8. A composition as claimed in claim 1 wherein, the living system are bacteria, fungi or any living cells.

9. A composition as claimed in claim 1 wherein the concentration of the cow urine *(Go-mutra)* distillate is in the range between 0.001 µl/ml to 100 µl/ml.

10. A process of preparing powder (Gm-IV) from cow urine distillate, said process comprising:
a mixing cow urine distillate with half the volume of methanol and extracting with hexane.
b lyophilising the hexane fraction (Gm-I) and testing for similar activity as that of cow urine *(Go-mutra)*.
c extraction of the aqueous fraction with ethyl acetate and lyophilising the ethyl acetate fraction (Gm-II) and testing for similar activity as that of cow urine *(Go-mutra)*.
d further, extracting the aqueous fraction containing white precipitate with butanol and lyophilising the butanol fraction (Gm-III) having pale yellow precipitate and testing for similar activity as that of cow urine *(Go-mutra)*.
e drying the remaining aqueous fraction containing white crystalline precipitate (GM-IV) and testing for similar activity as that of cow urine *(Go-mutra)*.

11. A process as claimed in claim 10 wherein, the lyophilized extract Gm-IV from cow urine distillate is devoid of typical cow urine distillate smell.

12. A process as claimed in claim 10 wherein the lyophilized extract Gm-IV has the following properties.
Colour: White
Physical state: Solid Crystalline
Solubility: Water-soluble and mixtures containing water
Melting point: Above 400°C
Specific Gravity: 1.006
RF value in methanol: Chloroform (50:50) phase: 0.65

13. A process as claimed in claim 10 wherein, starting cow urine distillate has HPLC properties having two major peaks with retention time 5.334 and 11.310 min.

14. A process as claimed in claim 10 wherein, the lyophilized extract possess all the activities like that of the distillate at a concentration in the range of 0.1 to 100µg/ml, with much more stability at room temperature and solubility in water.

15. A lyophilized bio-active product obtained from cow urine distillate which has the following physical characters:
Colour: White
Physical State: Solid Crystalline
Solubility: Water-soluble and mixture containing water
Melting Point: Above 400°C
Specific Gravity: 1.006
RF value in methanol: Chloroform (50:50) phase: 0.65

16. A lyophilized product as claimed in claim 19 wherein, the bio-active product GM-IV is devoid of typical cow urine distillate smell, and/or wherein the bio-active product is used at concentration 0.1 to 100µg/ml, with much more stability at room temperature and solubility in water.

17. A lyophilized product as claimed in claim 19 is used to enhance membrane permeability of molecules, drugs across the semi-permeable membrane and mammalian gut membrane, and can be used for enhancing intestinal transport and transport of molecules across membranes of various biological functions.

18. A composition comprising an effective amount of bioactive lyophilized fraction obtained from cow urine distillate, which is used as a bio-enhancer and bioavailability facilitator and one or more nutraceuticals, antibiotic, anti-infective, anticancer agents.

19. A composition as claimed in claim 18 wherein the bioactive fraction has enhancing activity of anti-bacterial agents, anti-cancer agents and anti-tuberculosis agents by 2 to 80 fold, or of anti-tuberculosis agents or anti-cancer agents by 2 to 20 fold.

20. A composition as claimed in claim 18 wherein the anti bacterial agents are selected from but not limited to the group comprising quinolones, rifampicin, tetracycline and ampicillin, and anti-tuberculosis agents selected from isoniazid, pyrazinamide and ethambutol.

21. A composition as claimed in claim 20 wherein the anti-cancer agent is paclitaxel (Taxol).

22. Use of cow urine distillate in the manufacture of a medicament for use as a bio-enhancer and bioavailability facilitator of one or more nutraceuticals, antibiotics, anti-infective and anticancer agents.

23. Use as claimed in claim 22 wherein the anti bacterial agents are selected from the group comprising quinolones, rifampicin, tetracycline and ampicillin; or wherein the anti bacterial agent is an anti-tuberculosis agent selected from isoniazid, pyrazinamide and ethambutol.

24. Use as claimed in claim 23 wherein the anti-cancer agent is paclitaxel (Taxol).

25. Use as claimed in claim 23 wherein the cow urine distillate is used as bioavailability facilitator for anticancer therapy directly or in combination with anticancer molecules.

26. Use as claimed in claims 22-25 wherein, the concentration of the cow urine distillate and lyophilized powder is very critical and increased amount above the upper limit may mar the effect/activity in formulation or administered dosage.

## Patentansprüche

1. Pharmazeutische Zusammensetzung umfassend eine wirksame Menge von Kuhham-Destillat als Bioverfügbarkeitsförderer und pharmazeutisch akzeptable Zusatzmittel ausgewählt aus antineoplastischen Verbindungen, Antibiotika, Medikamenten, therapeutischen und nutrazeutischen Mitteln, Ionen und ähnlichen Molekülen, die gezielt auf Lebendsysteme angewandt werden.

2. Zusammenfassung nach Anspruch 1, wobei das Kuhham-Destillat als Bioverfügbarkeitsförderer für die antineoplastische Therapie direkt oder in Kombination mit antineoplastischen Molekülen, bei der TB-Therapie einschließlich der Vielfachresistenz-Tuberkulose, in Kombination mit Isoniazid und anderen Antituberkulose-Mitteln oder bei der Antifungaltherapie für Pilzinfektionen verwendet wird.

3. Zusammensetzung nach Anspruch 1, wobei der Bioverfügbarkeitsförderer zum Übertragen der Verbindung durch die Membran und zur besseren Wirksamkeit an der Zielstelle beiträgt und/oder das Antibiotikum und andere Moleküle dabei unterstützt, am Target durch Erhöhen der Wirksamkeit besser zu wirken und/oder die Membrandurchlässigkeit für Moleküle, Medikamente durch die halbdurchlässige Membran und Säugerdarmmembran verbessert und zum Verbessern des Transports durch den Darm und des Transports von Molekülen durch Membranen verschiedener biologischer Funktionen verwendet wird.

4. Zusammensetzung nach Anspruch 1, wobei es sich bei den Antibiotika um Fluorchinolone wie Nalidixinsäure und andere wie Rifampicin, Tetracyclin und Ampicillin handelt, diese jedoch nicht darauf beschränkt sind.

5. Zusammensetzung nach Anspruch 1, wobei die Antibiotika, Ionen und ähnlichen Verbindungen Isoniazid und Wasserstoffperoxid sind.

6. Zusammensetzung nach Anspruch 1, wobei die antifungalen Mittel Azole, Clotrimazol, Mystatin, Amphotericin und ähnliche Materialien sind.

7. Zusammensetzung nach Anspruch 1, wobei Pilze umfassende Infektionen, Myzeten, Candida, Hefe- und andere fungizide Verbindungen sind.

8. Zusammensetzung nach Anspruch 1, wobei es sich bei dem Lebendsystem um Bakterien, Pilze oder irgendwelche Lebendzellen handelt.

9. Zusammensetzung nach Anspruch 1, wobei die Konzentration des Kuhharn- (Go-*mutra-*) Destillats im Bereich von 0,001 µl/ml bis 100 µl/ml liegt.

10. Verfahren für das Zubereiten von Pulver (Gm-IV) aus Kuhham-Destillat, wobei das Verfahren folgendes umfasst:
a) Mischen des Kuhharn-Destillats mit der Hälfte des Volumens an Methanol und Extrahieren mit Hexan.
b) Lyophilisieren der Hexanfraktion (Gm-I) und Prüfen auf ähnliche Aktivität wie diejenige von Kuhham (Go-*mutra*).
c) Extrahieren der wässrigen Fraktion mit Ethylacetat und Lyophilisieren der Ethylacetatfraktion (Gm-II) und Prüfen auf ähnliche Aktivität wie diejenige von Kuhham (Go-*mutra*).
d) Des Weiteren Extrahieren der wässrigen, weißen Niederschlag enthaltenden Fraktion mit Butanol und Lyophilisieren der hellgelben Niederschlag enthaltenden Butanolfraktion (Gm-III) und Prüfen auf ähnliche Aktivität wie diejenige von Kuhharn (Go-*mutra*).
e) Trocknen der verbleibenden wässrigen, weißen kristallinen Niederschlag enthaltenden Fraktion (Gm-IV) und Prüfen auf ähnliche Aktivität wie diejenige von Kuhharn (Go*-mutra*).

11. Verfahren nach Anspruch 10, wobei das lyophilisierte Extrakt Gm-IV aus Kuhham-Destillat nicht den typischen Geruch von Kuhham-Destillat aufweist.

12. Verfahren nach Anspruch 10, wobei das lyophilisierte Extrakt Gm-IV folgende Eigenschaften aufweist:
Farbe: Weiß
Physikalischer Zustand: Kristallinischer Feststoff
Löslichkeit: Wasserlöslich und wasserhaltige Mischungen
Schmelzpunkt: über 400°C
Spezifisches Gewicht: 1,006
RF-Wert in Methanol: Chloroform (50:50) Phase: 0,65

13. Verfahren nach Anspruch 10, wobei das Anfangskuhham-Destillat HPLC-Eigenschaften mit zwei Hauptpeaks bei Retentionszeiten von 5,334 und 11,310 min aufweist.

14. Verfahren nach Anspruch 10, wobei das lyophilisierte Extrakt alle die Aktivitäten aufweist wie diejenigen des Destillats in einer Konzentration im Bereich von 0,1 bis 100 µg/ml, mit einer viel höheren Beständigkeit bei Raumtemperatur und Löslichkeit im Wasser.

15. Lyophilisiertes bioaktives Produkt, das aus Kuhham-Destillat erhalten wird und den folgenden physikalischen Charakter aufweist:
Farbe: Weiß
Physikalischer Zustand: Kristallinischer Feststoff
Löslichkeit: Wasserlöslich und wasserhaltige Mischung
Schmelzpunkt: Über 400°C
Spezifisches Gewicht: 1,006
RF-Wert in Methanol: Chloroform (50:50) Phase: 0,65

16. Lyophilisiertes Produkt nach Anspruch 19, wobei das bioaktive Produkt GM-IV nicht den typischen Geruch von Kuhham-Destillat aufweist und/oder wobei das bioaktive Produkt in einer Konzentration von 0,1 bis 100 µg/ml verwendet wird und eine viel höhere Beständigkeit bei Raumtemperatur und Löslichkeit in Wasser aufweist.

17. Ein lyophilisiertes Produkt nach Anspruch 19 wird zum Verbessern der Membrandurchlässigkeit für Moleküle, Arzneimittel durch die halbdurchlässige Membran und die Darmmembran von Säugern verwendet und kann zum Verbessern des Transports durch den Darm und des Transports von Molekülen durch Membranen mit verschiedenen biologischen Funktionen verwendet werden.

18. Zusammensetzung umfassend eine wirksame Menge der aus Kuhharn-Destillat erhaltenen bioaktiven lyophilisierten Fraktion, die als Bioverstärker und Bioverfügbarkeitsförderer verwendet wird sowie ein oder mehrere nutrazeutische Mittel, Antibiotika, Antiinfektions-, antineoplastische Mittel.

19. Zusammensetzung nach Anspruch 18, wobei die bioaktive Fraktion eine verbessernde Aktivität für antibakterielle Mittel, antineoplastische Mittel und Antituberkulosemittel um das 2 bis 80-fache oder für Antituberkulosemitteln oder von antineoplastischen Mitteln um das 2 bis 20-fache aufweist.

20. Zusammensetzung nach Anspruch 18, wobei die antibakteriellen Mittel aus der Gruppe ausgewählt werden umfassend Chinolone, Rifampicin, Tetracyclin und Ampicillin und Antituberkulosemittel, die unter Isoniazid, Pyrazinamid und Ethambutol ausgewählt werden, jedoch nicht darauf beschränkt sind.

21. Zusammensetzung nach Anspruch 20, wobei das antineoplastische Mittel Paclitaxel (Taxol) ist.

22. Verwendung von Kuhharn-Destillat bei der Herstellung eines Medikaments für die Verwendung als Bioverstärker und Bioverfügbarkeitsförderer für ein oder mehrere nutrazeutische Mittel, Antibiotika, anti-infektiöse und antineoplastische Mittel.

23. Verwendung nach Anspruch 22, wobei die antibakteriellen Mittel aus der Gruppe ausgewählt werden umfassend Chinolone, Rifampicin, Tetracyclin und Ampicillin oder wobei das antibakterielle Mittel unter Isoniazid, Pyrazinamid und Ethambutol ausgewählt wird.

24. Verwendung nach Anspruch 23, wobei das antineoplastische Mittel Paclitaxel (Taxol) ist.

25. Verwendung nach Anspruch 23, wobei das Kuhharn-Destillat als Bioverfügbarkeitsförderer für die antineoplastische Therapie direkt oder in Kombination mit antineoplastischen Molekülen verwendet wird.

26. Verwendung nach Ansprüchen 22-25, wobei die Konzentration des Kuhharn-Destillats und lyophilisierten Pulvers äußerst kritisch ist und eine erhöhte Menge über der oberen Grenze die Wirkung/Aktivität in Rezepturen oder verabreichten Dosen negativ beeinflussen kann.

## Revendications

1. Composition pharmaceutique comprenant une quantité efficace de distillat d'urine de vache comme promoteur de biodisponibilité et d'additifs acceptables du point de vue pharmaceutique, sélectionnés parmi les composés anticancéreux, les antibiotiques, les médicaments, les agents thérapeutiques et nutraceutiques, les ions et molécules similaires, ciblés sur les systèmes vivants.

2. Composition selon la revendication 1, dans laquelle le distillat d'urine de vache est utilisé comme promoteur de biodisponibilité pour la thérapie anticancéreuse directement ou en combinaison avec des molécules anticancéreuses ; dans la thérapie TB englobant la tuberculose résistante à de nombreux médicaments en combinaison avec l'isoniazide et d'autres agents anti-tuberculeux ; ou bien dans la thérapie antifongique pour les infections fongiques.

3. Composition selon la revendication 1, dans laquelle le promoteur de biodisponibilité contribue au transfert du composé à travers la membrane et à renforcer son efficacité sur le site cible ; et/ou aide les antibiotiques et autres molécules à mieux agir sur la cible en augmentant leur efficacité ; et/ou améliore la perméabilité de la membrane en stimulant le passage des molécules et médicaments à travers la membrane semi-perméable et la membrane de boyau de mammifère, et sert à améliorer le transport intestinal et le transport de molécules à travers les membranes de diverses fonctions biologiques.

4. Composition selon la revendication 1, dans laquelle les antibiotiques sont, mais sans s'y limiter, des fluoroquinolones comme l'acide nalidixique et d'autres comme la rifampicine, la tétracycline et l'ampicilline.

5. Composition selon la revendication 1, dans laquelle les antibiotiques, les ions et composés similaires sont l'isoniazide et le peroxyde d'hydrogène.

6. Composition selon la revendication 1, dans laquelle les antifongiques sont des azoles, le clotrimazole, la mystatine, l'amphotéricine et matériaux similaires.

7. Composition selon la revendication 1, dans laquelle les champignons recouvrant les infections fongiques sont mycétiques, la Candida, la levure ou d'autres composés fongicides.

8. Composition selon la revendication 1, dans laquelle le système vivant peut être des bactéries, des champignons ou toute cellule vivante.

9. Composition selon la revendication 1, dans laquelle la concentration du distillat d'urine de vache (*Go-mutra*) est comprise entre 0,001 µl/ml et 100 µl/ml.

10. Procédé de préparation de poudre (Gm-IV) à partir de distillat d'urine de vache, ledit procédé comprenant les phases suivantes :
a mélange de distillat d'urine de vache avec la moitié du volume de méthanol et extraction avec de l'hexane.
b lyophilisation de la fraction hexane (Gm-I) et recherche d'une activité similaire à celle de l'urine de vache (*Go-mutra*).
c extraction de la fraction aqueuse avec de l'acétate d'éthyle et lyophilisation de la fraction d'acétate d'éthyle (Gm-II), puis recherche d'une activité similaire à celle de l'urine de vache (*Go-mutra*).
d ensuite, extraction de la fraction aqueuse contenant un précipité blanc avec du butanol et lyophilisation de la fraction butanol (Gm-III) ayant un produit de précipitation jaune pâle, puis recherche d'une activité similaire à celle de l'urine de vache (*Go-mutra*).
e séchage de la fraction aqueuse restante contenant un précipité blanc cristallin (Gm-IV), puis recherche d'une activité similaire à celle de l'urine de vache (*Go-mutra*).

11. Procédé selon la revendication 10, dans lequel l'extrait lyophilisé Gm-IV à partir de distillat d'urine de vache est dépourvu de l'odeur typique du distillat d'urine de vache.

12. Procédé selon la revendication 10, dans lequel l'extrait lyophilisé Gm-IV a les propriétés suivantes :
Couleur : blanche
Etat solide : solide cristallin
Solubilité : soluble dans l'eau et mélange contenant de l'eau
Point de fusion : supérieur à 400°C
Poids volumique : 1,006
Valeur RF dans le méthanol : chloroforme (50:50) phase : 0,65

13. Procédé selon la revendication 10, dans lequel le distillat d'urine de vache présente des propriétés HPLC avec deux pics majeurs d'un temps de rétention de 5,334 et de 1,310 minutes.

14. Procédé selon la revendication 10, dans lequel l'extrait lyophilisé possède toutes les activités comme celles du distillat à une concentration comprise entre 0,1 et 100 µg/ml, avec une stabilité beaucoup plus grande à la température ambiante et une meilleure solubilité dans l'eau.

15. Produit bioactif lyophilisé obtenu à partir de distillat d'urine de vache ayant les caractéristiques physiques suivantes :
Couleur : blanche
Etat solide : solide cristallin
Solubilité : soluble dans l'eau et mélange contenant de l'eau
Point de fusion : supérieur à 400°C
Poids volumique : 1,006
Valeur RF dans le méthanol : chloroforme (50:50) phase : 0,65

16. Produit lyophilisé selon la revendication 15, dans lequel le produit bioactif Gm-IV est dépourvu de l'odeur typique du distillat d'urine de vache, et/ou dans lequel le produit bioactif est utilisé à une concentration comprise entre 0,1 et 100 µg/ml, avec une stabilité beaucoup plus grande à la température ambiante et une meilleure solubilité dans l'eau.

17. Produit lyophilisé selon la revendication 15, utilisé pour améliorer la perméabilité de la membrane en stimulant le passage des molécules et médicaments à travers la membrane semi-perméable et la membrane de boyau de mammifère, et sert à améliorer le transport intestinal et le transport de molécules à travers les membranes de diverses fonctions biologiques.

18. Composition comprenant une quantité efficace de fraction lyophilisée bioactive obtenue à partir de distillat d'urine de vache, utilisée comme biostimulant et promoteur de biodisponibilité et un ou plusieurs agents nutraceutiques, antibiotiques, anti-infectieux et anticancéreux.

19. Composition selon la revendication 18, dans laquelle la fraction bioactive présente une activité renforcée des agents antibactériens, des agents anticancéreux et des agents antituberculeux d'un facteur de 2 à 80, ou des agents antituberculeux ou des agents anticancéreux d'un facteur de 2 à 20.

20. Composition selon la revendication 18, dans laquelle les agents antibactériens sont sélectionnés dans le groupe comprenant les quinonoles, la rifampicine, la tétracycline et l'ampicilline, et les agents antituberculeux sélectionnés parmi l'isoniazide, le pyrazinamide et l'éthambutol.

21. Composition selon la revendication 20, dans laquelle l'agent anticancéreux est le paclitaxel (Taxol).

22. Utilisation du distillat d'urine de vache dans la fabrication d'un médicament pour utilisation comme biostimulant et promoteur de biodisponibilité et un ou plusieurs agents nutraceutiques, antibiotiques, anti-infectieux et anticancéreux.

23. Utilisation selon la revendication 22, dans laquelle les agents antibactériens sont sélectionnés dans le groupe comprenant les quinonoles, la rifampicine, la tétracycline et l'ampicilline, ou dans laquelle l'agent antibactérien est un agent antituberculeux sélectionné parmi l'isoniazide, le pyrazinamide et l'éthambutol.

24. Utilisation selon la revendication 23, dans laquelle l'agent anticancéreux est le paclitaxel (Taxol).

25. Utilisation selon la revendication 23, dans laquelle le distillat d'urine de vache s'utilise comme promoteur de biodisponibilité pour la thérapie anticancéreuse directement ou en combinaison avec des molécules anticancéreuses.

26. Utilisation selon les revendications 22 à 25, dans laquelle la concentration de distillat d'urine de vache et de poudre lyophilisée est très critique et une quantité dépassant la limite supérieure risque de compromettre l'efficacité/l'activité dans la formulation ou le dosage administré.
